Europäisches Patentamt

(19) European Patent Office

Office européen des brevets

(11) Publication number: **0 009 858**

**A1**

## (12) EUROPEAN PATENT APPLICATION

(21) Application number: 79301002.6

(22) Date of filing: 30.05.79

(51) Int. Cl.³: **A 61 K 9/16**

(30) Priority: 02.10.78 GB 3900778

(43) Date of publication of application:
16.04.80 Bulletin 80/8

(84) Designated Contracting States:
AT BE CH DE FR IT NL SE

(71) Applicant: SPEYWOOD LABORATORIES LTD.
Chancel House Bingham
Nottingham NG13 8DR(GB)

(72) Inventor: Heath, David
Granby House
Granby, Nottingham(GB)

(72) Inventor: Lees, Peter
The Cottages Main Street
Cropwell Butler Nottingham(GB)

(74) Representative: Collier, Jeremy Austin Grey et al,
J.A.Kemp & Co. 14, South Square Gray's Inn
London WC1R 5EU(GB)

(54) Gum granulates, and their preparation and use.

(57) Granulates of guar gum, locust bean gum, pectin, or a water-soluble non-toxic alkylcellulose consisting of particles having sizes within the range 100 to 1000 $\mu$ with less than 5% by weight of the particles having a size below the lower limit and having a water content of 5 to 25% by weight are useful for oral administration to patients to lower cholesterol and/or glucose levels in the blood. They can be dispersed in water to give easily drinkable mixtures.

EP 0 009 858 A1

Croydon Printing Company Ltd.

# DESCRIPTION

## "GUM GRANULATES, AND THEIR PREPARATION AND USE"

The present invention relates to a new form of gums, their preparation and use.

It is known (Jenkins et al, The Lancet 1975, 1116 and 1977, 779) that certain gums, e.g. guar and pectin, when administered to patients in substantial quantities have significant lowering effects on the cholesterol and glucose levels in the blood. These substances can, therefore, be used as medicaments for the control of carbohydrate metabolism and especially in the treatment of diseases like hyperglycaemia (including hyperglycaemia associated with diabetes), hyperlipoproteinaemia and obesity.

However, the desired effects only occur when substantial quantities (e.g. 4 to 6 g 3 to 5 times a day, or about 10-30 g per day) are taken by the patient and this presents a major practical problem because the gums are extremely hydrophilic and tend to gel very quickly in contact with water thus making it impossible to administer them in their presently known form to patients either by themselves or in admixture with, for example, foodstuffs or drinks.

If gums in their commercially available form (a finely divided powder) are added to water with inadequate mixing, sticky lumps are formed which then dissolve slowly in water and are difficult and distasteful to consume. Alternatively, if the mixing is adequate, a very viscous solution is very rapidly formed which is unpleasant to drink and can be unacceptable to many patients.

Adding powdered gum to foodstuffs is possible but is inconvenient and liable to cause an unpleasant gel to be formed; moreover, control of the amount consumed is in this case difficult.

The present invention provides a new form of gums which surprisingly can be dispersed in water to give a solution of moderate viscosity which can easily be drunk.

This new form of gums is a granulate of guar gum, locust bean gum, pectin, or a water-soluble non-toxic alkyl-cellulose, e.g. methyl cellulose, consisting of particles having sizes within the range 100 to 1000 µ, preferably 125 to 500 µ or better 300 to 710 µ, with less than 5% by weight of the particles having a size below the lower limit and having a water content of 5 to 25%, preferably 9-11%, by weight.

This gum granulate mixes easily with water or any other drink like fruit juice, milk, coffee, or tea to give a low viscosity dispersion which can easily be drunk by the patient.

It will be understood that while the gum must not form a highly viscous solution before it is drunk, it is necessary that it shall gel completely soon after consumption so that the desired effects may be obtained. In practical terms, this means that the granulates of the invention must not form, when mixed with water, a thick gel in under 3 minutes but should form such a gel in less than about 5 minutes, i.e. shortly after consumption. The granulates of the invention as defined above satisfy this condition.

According to another aspect of the invention there is provided a method of preparing a gum granulate of the invention which comprises agglomerating a powder of the said gum having an average particle size below 100 µ by treating 1 part by weight of the said powder under vigorous agitation with 0.075 to 0.4 part by weight of water and then (if necessary) drying the gum granulate obtained to a final water content of 5 to 25% by weight. Preferably 0.1 to 0.25 part by weight of water is added to 1 part of gum.

The water-treatment is preferably accomplished by spraying the water in fine droplets onto the agitated gum powder or by blowing water vapour through the agitated gum powder.

Should the product contain any particles of undesired particle size (too big or too small), such particles are removed by sieving, though, as already indicated, it is permissible to allow a maximum of 5% of particles under the specified minimum size to remain.

Admixtures of two or more gums can be used if desired. The water treatment and the drying can be performed with conventional equipment provided that the gum powder is treated with very fine particles of water or with water vapour and that the gum powder is agitated, e.g. with a high speed horizontal or vertical mixing machine.

The process can be performed continuously or batch-wise, or one step (granulation or drying) can be performed continuously and the other batch-wise.

It is surprising that in the process of the invention no gel is formed but a crumbly granulate which surprisingly when mixed with water (approximately 5 g with 100 to 200 ml of water) does not give immediately a gel or lumps which are wetted only on the surface and are still dry inside, but forms a dispersion which is easy to drink.

The water-miscible gum granulate of the present invention is nearly tasteless and can be administered

- 4 -

to patients as an aqueous dispersion. Of course other
ingredients such as flavours, colours, diluents, and
lubricants can be added if desired. The daily dose can
be, as already mentioned, 3 to 5 times 4 to 6 g, especially
5 times a standard dose of 5 g of gum, which can be
supplied in a sachet. The gum granulate according to this
invention can also be mixed with other substances and in
particular with foodstuffs. It is also possible to make
up the granulate into tablets containing a predetermined
weight of the gum. Such tablets disintegrate readily
in water especially if they are made to be effervescent.
Flavours and colours may, of course, be added as desired.

The following Examples illustrate the invention.

EXAMPLE 1

50.0 Kg of commercial guar powder are charged
to a high speed horizontal mixing machine and a fine
jet of 10 kg water is sprayed onto the moving powder
during 40 minutes.

The resulting granules are then spread onto
baking-trays at a depth of no more than 1 cm and heated
to 195°C for 12 minutes. The granules are allowed to
cool, and then sieved. Granules greater than 500 $\mu$ and
granules smaller than 125 $\mu$ are rejected. Slightly better
results are obtained if the sieving is such as to reject
granules greater than 710 $\mu$ or less than 300 $\mu$. The
remaining granules are collected and filled in sachets
each containing 5 g of guar.

The rejected fractions of the granulate,
optionally after grinding, can be added to the starting
material of the process.

EXAMPLE 2

Guar gum, at the rate of 445 kg per hour is fed
into a vertical fan mixer (Schugi) and atomised water,
at 83 kg per hour, is sprayed into the rapidly agitated

- 4 -

guar as it falls through the mixer. The resulting granules fall directly into a fluid bed drier which operates at an air temperature of 130°C (granule temperature approximately 55°C). The capacity of the fluid bed drier is such as to allow a continuous process, i.e. the dry granules (containing 10-12% $H_2O$) are taken from the drier at the same rate as the guar powder is introduced into the vertical fan mixer. The granules are allowed to cool and then sieved. Granules greater than 500μ and most or all of the granules smaller than 125μ are rejected (so that the product contains not more than 5% of particles below 125μ). The remaining granules, between 125 and 500μ, are collected and filled in sachets each containing 5 g. of guar.

The granules less than 125μ are added to the starting material for recycling, and the granules greater than 500μ are ground to a fine powder before recycling.

As in Example 1, it has been found that slightly better results are obtained if the sieving is such as to reject granules greater than 710μ and smaller than 300μ, not more than 5% of particles below 300μ being retained. The rejected granules can be re-used as before.

The granulator and/or the drier can if desired be run on a batch basis.

## C L A I M S

1. A granulate of a gum which is guar gum, locust bean gum, pectin, or a water-soluble non-toxic alkyl-cellulose consisting of particles of gum having sizes within the range 100 to 1000µ, with less than 5% by weight of the particles having a size below the lower limit and having a water content of 5 to 25% by weight.

2. A granulate according to claim 1 consisting of particles having sizes within the range 125 to 500µ and having a water content of 9 to 11% by weight.

3. A granulate according to claim 1 consisting of particles having sizes within the range 300 to 710µ and having a water content of 9 to 11% by weight.

4. A granulate according to any of the preceding claims in which the gum is guar gum.

5. Process for the production of a gum granulate as claimed in claim 1 which comprises agglomerating a powder of the said gum having an average particle size below 100µ by treating 1 part by weight of the said powder under vigorous agitation with 0.075 to 0.4 part by weight of water and then, if necessary, drying the gum granulate obtained to a final water content of 5 to 25% by weight.

6. Process according to claim 5 in which 0.1 to 0.25 part by weight of water is used per part by weight of gum.

7. Process according to claim 5 or 6 in which the granulate obtained consists of particles having sizes within the range 125 to 500μ and having a water content of 9 to 11% by weight.

8. Process according to claim 5 or 6 in which the granulate obtained consists of particles having sizes within the range 300 to 710μ and having a water content of 9 to 11% by weight.

9. Process according to any of claims 5 to 8 in which the gum is guar gum.

10.A granulate as claimed in any of claims 1 to 4 for use in therapy to lower the cholesterol and/or glucose level in the blood.

## DOCUMENTS CONSIDERED TO BE RELEVANT

| Category | Citation of document with indication, where appropriate, of relevant passages | Relevant to claim |
|---|---|---|
| | US - A - 3 313 800 (G.J. JACKSON) | 1,4,10 |
| | * Claims; examples; column 2, lines 52-61; column 1, lines 14-31; column 1, lines 52-64 * | |
| | -- | |
| | US - A - 3 415 927 (I.S. BUTENSKY) | 1,4,10 |
| | * Claims; examples; column 1, line 25 to column 2, line 8 * | |
| | -- | |
| | DE - A - 2 128 378 (MYLAENS ARMIN) | 1,10 |
| | * Claims; page 3, paragraph 2 to page 4, paragraph 1 * | |
| | -- | |
| | DE - A - 2 518 270 (SHINETSU CHEMICAL) | 1,5,6 |
| | * Claims; page 1, paragraph 1; page 3, paragraph 3 to page 5, paragraph 1; page 7, paragraph 3 to page 8, paragraph 2; examples * | |
| | -- | |
| | DE - A - 2 620 456 (ENTEROFAGOS) | 1 |
| | * Claims * | |
| | ---- | |

**CLASSIFICATION OF THE APPLICATION (Int. Cl. 3)**

A 61 K 9/16

**TECHNICAL FIELDS SEARCHED (Int.Cl. 3)**

A 61 K 9/16

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 16-01-1980 | VANHECKE |

EPO Form 1503.1 06.78